# EUROPEAN PATENT SPECIFICATION

(11) **EP 4 260 044 B1**
(45) Date of publication and mention of the grant of the patent: **01.10.2025**
(21) Application number: 21827216.9
(22) Date of filing: 25.11.2021
(51) Int. Cl.: G01N 9/02, G01N 33/02

(54) **METHOD, COMPUTER PROGRAM, COMPUTER SYSTEM AND ASSEMBLY FOR THE NON-DESTRUCTIVE DETERMINATION OF THE JUICE CONTENT OF JUICE FRUITS, AS WELL AS THE USE OF THIS ASSEMBLY FOR THE QUALITY CLASSIFICATION OF JUICE FRUITS**
VERFAHREN, COMPUTERPROGRAMM, COMPUTERSYSTEM UND ANORDNUNG ZUR ZERSTÖRUNGSFREIEN BESTIMMUNG DES SAFTGEHALTES VON FRÜCHTEN, SOWIE VERWENDUNG DIESER ANORDNUNG ZUR QUALITÄTSKLASSIFIZIERUNG VON FRÜCHTEN
PROCÉDÉ, PROGRAMME D'ORDINATEUR, SYSTÈME INFORMATIQUE ET ENSEMBLE POUR LA DÉTERMINATION NON DESTRUCTIVE DE LA TENEUR EN JUS DE FRUITS À JUS, AINSI QUE L'UTILISATION DE CET ENSEMBLE POUR LA CLASSIFICATION DE QUALITÉ DE FRUITS À JUS

(30) Priority: 11.12.2020 IT 202000030629
(43) Date of publication of application: 18.10.2023
(73) Proprietor: ALL CITRUS S.R.L., 95128 Catania (CT) (IT)
(72) Inventor: CAMPAGNARO, Daniele, 45020 Lusia (RO) (IT); MODICA, Nicola, 35031 Abano Terme(PD) (IT); GAETAN, Carlo, 31033 Castelfranco Veneto (TV) (IT); GIRARDI, Paolo, 35043 Monselice (PD) (IT); RIELLO, Pietro, 35132 Padova (PD) (IT)
(74) Representative: Autuori & Partners S.R.L.
(86) International application number: PCT/IB2021/060981
(87) International publication number: WO 2022/123385

(56) References cited:
- EP-A2- 0 572 341
- CN-A- 108 057 638
- ARENDSE E. ET AL: "Non-destructive estimation of pomegranate juice content of intact fruit using X-ray computed tomography", PROCEEDINGS OF THE FOURTH INTERNATIONAL DATE PALM CONFERENCE : ABU DHABI, UNITED ARAB EMIRATES, MARCH 15 - 17, 2010, no. 1201, 1 April 2018 (2018-04-01), BE, pages 297 - 302, XP055827830, ISBN: 978-90-6605-633-6, Retrieved from the Internet <URL:http://dx.doi.org/10.17660/ActaHortic.2018.1201.40> DOI: 10.17660/ActaHortic.2018.1201.40

## Description

### Field of the invention

The present invention relates to the technical field of agri-food, and it particularly relates to an assembly, a method, a software and a computer system for the non-destructive determination of juice content of juice fruits.

The invention also relates to the use of the aforementioned assembly for the quality classification of juice fruits.

### Definitions

In the present document, the expression "juice fruit" or derivatives is used to indicate any fruit from which juice can be extracted.

In the present document, the expression "juice content" or derivatives is used to indicate the percentage of juice present in the juice fruit with respect to the total weight of the juice fruit.

In the present document, the expression "quality classification" or derivatives referring to a juice fruit is used to indicate the association of a certain characteristic, for example the percentage of juice contained therein, with such juice fruit.

### State of the Art

It is known that agri-food companies need to classify juice fruits, for example citrus fruits, according to the juice content.

From the regulatory point of view, EC Regulation n° 1221/2008 provides for a minimum amount of juice for citrus fruits intended for fresh delivery to the consumer. In the case of oranges, this juice content amounts to 30%.

To date, there are destructive methods which provide for the destruction of the fruit to determine the juice content thereof. These methods clearly cannot be implemented on an industrial scale.

On the other hand, apparatuses for the non-destructive measurement of dimensional characteristics of fruits in general by means of image analysis, are known. An example of such apparatuses is known from the document US5449911.

However, such types of apparatuses do not measure the juice content of the fruits. A method for non-destructive determination of of juice content in pomegranates is disclosed in "Non-destructive estimation of pomegranate juice content of intact fruit using X-ray computed tomography", Arendse E. et al; ACTA HORTICULTURAE, no. 1201, 1 April 2018 (2018-04-01), pages 297-302, BE ISSN: 0567-7572, DOI:10.17660/ActaHortic.2018.1201.4.

### Summary of the invention

The object of the present invention is to overcome the drawbacks outlined above by providing an assembly, a method and a computer program for the non-destructive determination of the juice content of juice fruits that is highly efficient and cost-effective.

A further object of the present invention is to provide an assembly, a method, a computer program and a computer system for the non-destructive determination of the juice content of juice fruits that allow the minimum waste of the batch of fruits to be measured and of resources used.

A further object of the present invention is to provide an assembly, a method, a computer program and a computer system for the non-destructive determination of the juice content of juice fruits which is extremely easy to implement even on existing processing lines.

These and other objects which will be more apparent hereinafter, are attained by a method, a computer program and a computer system for the non-destructive determination of the juice content of juice fruits, according respectively to claims 1, 7 and 9.

In a further aspect of the invention, an assembly for the non-destructive determination of the juice content of juice fruits and the use thereof for the quality classification of the juice fruits according respectively to claims 10 and 11.

In particular, the invention relates to a computer-implemented method for the non-destructive determination of the juice content of juice fruits, for example citrus fruits, comprising at least the following steps:
- collecting first data relating to the estimated volume and the actual weight of said at least one juice fruit;
- collecting at least one first plurality of pairs of reference data for said at least one juice fruit, each pair of reference data consisting of a reference density value and a corresponding value relating to the reference amount of juice;
- calculating, based on the first data, the density of the at least one juice fruit;
- processing, based on the reference data and the calculated density value, a value relating to an estimated amount of juice for the at least one juice fruit, for example the percentage by weight of juice estimated with respect to the weight of the at least one fruit.

Therefore, this will allow to determine the juice content of the juice fruits in a non-destructive manner. As a matter fact, for each fruit this juice content will be determined simply by estimating the volume thereof, for example through an integrated electronic system of video cameras of the known type, capable of determining the shape and size of the fruit, and weighing it, for example by means of a weighing scale.

Therefore, this will allow to obtain the density thereof in a per se known manner and to compare this datum with the reference ones, in which each density value corresponds to a determined average juice content.

The reference data may be data already available and implemented in the computer, for example data relating to juice fruits of a predetermined known quality, or they will be obtained starting from a predetermined batch.

To this end, it may be possible to select a predetermined number of sample fruits, for example 40 - 80 fruits of the batch, and - for each- carry out the steps of:
- collecting second data relating to the estimated volume, to the actual weight and to the amount of juice extracted therefrom;
- calculating, based on the estimated volume and actual weight values, the density;
- calculating, based on the values of estimated volume and/or actual weight and of the amount of juice extracted, a value relating to the amount of juice extracted, preferably the percentage by weight with respect to the actual weight of the fruit;
- associating - with the calculated density - the calculated value relating to the extracted amount of juice to create a pair of calibration data.

The actual weight and the estimated volume of the sample fruits can be obtained as described above, while the juice can be obtained by means of appropriate extraction means of the known type, for example a professional citrus squeezer of the known type, suitable to extract the juice from the fruit by squeezing it against a rotating shaped pin, and then measured using measuring means which vary depending on whether the value to be obtained is linked to the weight, to the volume or to a different parameter of the extracted juice.

In the preferred but not exclusive embodiment of the invention, in which the value relating to the estimated amount of juice of the fruit is the percentage by weight with respect to the weight of the fruit, the measuring means may consist of a scale for weighing the extracted juice.

The method according to the invention may therefore provide for the steps of:
- collecting third data relating to all pairs of calibration data relating to sample juice fruits;
- calculating the linear regression of this calibration data to obtain the reference data of the batch from which the sample fruits were taken.

The above allows to determine the juice content of any batch of juice fruits in a simple, practical and precise manner, minimising the waste of fruits.

The method is extremely easy to implement, even on existing processing lines.

This method may be useful, for example, for classifying juice fruits depending on the estimated juice content, for example classifying citrus fruits depending on whether the juice content is greater or lesser than the aforementioned EC standard.

In a further aspect of the invention, there may be provided for a computer program for implementing the aforementioned method, as well as a computer system that runs such computer program and an assembly including such computer system.

In a further aspect of the invention, there may be provided for the use of this assembly for the quality classification of juice fruits based on the estimated juice content.

The dependent claims define advantageous embodiments of the invention.

### Brief description of the drawings

Further characteristics and advantages of the invention will be more apparent in light of the detailed description of some preferred but non-exclusive embodiments of the invention, illustrated by way of non-limiting example with reference to the attached drawings, wherein:
**FIG. 1** is a schematic view of the orange classification process **F;**
**FIG. 2** is a schematic view of the operation of the computer system **50;**
**FIG. 3** is a schematic view of the process for calibrating the reference curve relating to oranges **F** belonging to a batch **LF.**

### Detailed description of some preferred embodiments

With reference to the mentioned figures, herein described is an assembly **1** for the non-destructive determination of the juice content, for example oranges **F.** Although hereinafter reference will be made to the latter for the sake of simplicity, it is clear that the juice fruits may vary without departing from the scope of protection of the attached claims.

The assembly **1** may comprise a machine **5** with an inlet **10** and an outlet **40** for oranges **F.** Although hereinafter reference will be made to the latter for the sake of simplicity, it is clear that the number of inlets and outlets may vary without departing from the scope of protection of the attached claims.

Scanning means **20,** which may be made as disclosed by the document US5449911, and weighing means **30,** for example a load cell, may be interposed between the inlet **10** and the outlet **40.**

In a per se known manner, the scanning means **20** may determine the estimated volume **VsF** of the oranges **F,** while the weighing means **30** may determine the actual weight **PrF** thereof.

It is clear that even if the present document discloses a machine which includes both scanning and weighing means, the latter may be separated from each other without departing from the scope of protection of the attached claims.

The assembly **1** may further comprise a computer system **50,** which may be operatively connected to the scanning means **20** and to the weighing means **30** to collect the data relating to the estimated volume **VsF** and to the actual weight **PrF** of the oranges **F.**

The operative connection may vary, for example of the physical type wired or through WiFi or similar networks. The operative connection may also be indirect, for example the data of the scanning and weighing means may be stored in a physical unit or cloud which in turn can be operatively connected to the computer system **50.**

Essentially, the computer system may include a data collection unit **51** and a microprocessor unit **52,** which - in a preferred but non-exclusive embodiment - may be contained in a single PLC unit **53.**

However, it is clear that even if the present document discloses a single PLC unit which includes both the data collection unit **51** and the microprocessor unit **52,** the latter may be separated from each other without departing from the scope of protection of the attached claims. For example, the data may be stored in one or more databases in the cloud, and the microprocessor unit may be a PC or a laptop which can be connected to the cloud.

The data collection unit **51** may collect the data relating to the estimated volume **VsF** and the actual weight **PrF** of oranges **F** in any manner, whether manual or automatic. Besides this data, the data collection unit **51** may collect one or more plurality of pairs of reference data **Dr1, %r1; Dr2, %r2; Dr3, %r3...** regarding juice fruits.

The reference data may be present on a storage unit of the PLC **53** or it may be loaded from a cloud.

Suitably, each pair of reference data may consist of a reference density value **Dr1; Dr2; Dr3...** and a corresponding value relating to a reference amount of juice **%r1; %r2; %r3...,** for example, the percentage by weight of juice present in the fruit.

It is clear that even if the present document discloses a value relating to an amount of juice as the percentage by weight of juice present in a fruit, this value may vary without departing from the scope of protection of the attached claims.

Basically, the set of this data represents a curve having - in abscissa or ordinate - the density values and - in abscissa or ordinate - the percentage values of juice.

Such curve may be predetermined based on the characteristics of the fruit, for example quality and source, or it may be calibrated using the procedure described hereinafter
The microprocessor unit **52** may run a computer program, whether resident or cloud-based, which may calculate the density **DcF** of the orange **F** starting from the estimated volume and actual weight **VsF, PrF** data mentioned above and estimate the juice percentage **%sF** of the orange **F** by means of the aforementioned curve.

This allows to estimate - with reasonable certainty - the juice content of each orange **F** without damaging it.

The estimated juice percentage **%sF** of the orange **F,** as observed above, may be used for classifying oranges from a quality point of view. For example, according to the aforementioned EC standard, the oranges may be marketed fresh or not fresh depending on whether the estimated juice percentage **%sF** of the orange **F** is greater or lesser than 30%.

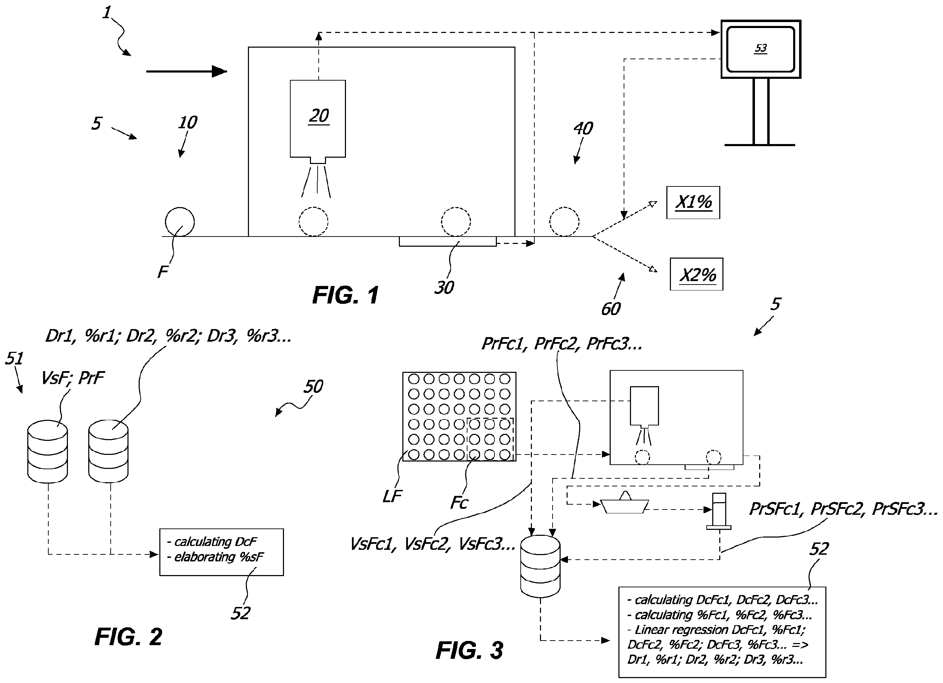

The selection may be carried out manually or, as illustrated in FIG. 1, automatically. In this case, the computer system **50** and means **60** for automatically diverting the orange **F** toward two or more containers **X1%, X2%** designed to contain oranges classified differently, for example oranges according to the EC standard mentioned, must be operatively connected directly or indirectly.

Should the reference data **Dr1, %r1; Dr2, %r2; Dr3, %r3...** be unknown, or in any case should one seek reference data relating to a predetermined batch of oranges **LF,** 40 - 80 sample oranges **Fc1, Fc2, Fc3...** may be taken from this batch and made to pass once or several times through scanning means **20** and weighing means **30.**

This will allow to obtain the estimated volume **VsFc1, VsFc2, VsFc3**...and actual weight **PrFc1, PrFc2, PrFc3...** values of the sample oranges **Fc.**

Then, the juice extracted using appropriate extraction means **70** may be extracted from each of the latter. The extracted juice may be then weighed to obtain the weight **PrSFc1, PrSFc2, PrSFc3....**

Then, the microprocessor unit **52** may run a sub-program for calculating - based on the aforementioned data - the density value **DcFc1, DcFc2, DcFc3...** and the juice percentage **%Fe1, %Fc2, %Fc3...** relating to each sample **Fc.**

This will allow to create a plurality of calibration data **DcFc1, %Fc1; DcFc2, %Fc2; DcFc3, %Fc3...** consisting of the calculated density pairs **DcFc1, DcFc2, DcFc3...** and percentage of juice **%Fc1, %Fc2, %Fc3...** extracted for each sample-orange **Fc.**

These data may be collected in the database **54,** and taken from here by the microprocessor unit **52** and used to calculate the linear regression thereof, so as to obtain the reference data **Dr1, %r1; Dr2, %r2; Dr3, %r3...** relating to the batch **LF.**

Therefore, the operations above may be repeated for each of the remaining oranges of this batch for example with the aim of classifying them according to the aforementioned EC standard.

Possibly, the microprocessor unit **52** may be configured so that - when determining the estimated volume **VsFc1, VsFc2, VsFc3**...and the actual weight **PrFc1, PrFc2, PrFc3...** of the sample oranges **Fc** - it discards the data relating to oranges having characteristics already measured previously. In this manner, the entire calibration process and subsequent sorting of the fruits will be fully automated, optimising work times and making the procedure easier to reproduce and objectively repeatable, eliminating the subjectivity of the measurements carried out manually.

In the light of the above, it is clear that the invention attains the pre-set objectives.

Even though the invention has been described with particular reference to the attached figures, the reference numerals used in the description and in the claims are meant for improving the intelligibility of the invention and thus do not limit the claimed scope of protection in any manner whatsoever.

## Claims

1. A computer-implemented method for the non-destructive determination of the juice content of at least one juice fruit, comprising at least the following steps:
- collecting first data relating to the estimated volume **(VsF)** and the actual weight **(PrF)** of said at least one juice fruit **(F);**
- collecting at least one first plurality of pairs of reference data **(Dr1, %r1; Dr2, %r2; Dr3, %r3...)** for said at least one juice fruit, each pair of reference data consisting of a reference density value **(Dr1; Dr2; Dr3...)** and a corresponding value relating to the reference amount of juice **(%r1; %r2; %r3...);**
- calculating, based on said first data **(VsF, PrF),** the density **(DcF)** of said at least one juice fruit **(F);**
- processing, based on said at least one first plurality of pairs of reference data **(Dr1, %r1; Dr2, %r2; Dr3, %r3...)** and on said calculated density value **(DcF),** a value relating to an estimated amount of juice **(%sF)** for said at least one of juice fruit **(F).**

2. Method according to claim 1, wherein said at least one juice fruit **(F)** is of a predetermined known quality, said at least one first plurality of pairs of reference data **(Dr1, %r1; Dr2, %r2; Dr3, %r3...)** relating to said predetermined known quality.

3. Method according to claim 1, suitable to determine the juice content of juice fruits **(F)** belonging to a predetermined batch **(LF),** said reference data **(Dr1, %r1; Dr2, %r2; Dr3, %r3...)** being determined starting from a predetermined number of sample juice fruits **(Fc1, Fc2, Fc3...)** belonging to said predetermined batch **(LF),** the method further comprising for each of said sample juice fruits **(Fc1, Fc2, Fc3**...) at least the following steps:
- collecting second data relating to the estimated volume **(VsFc1, VsFc2, VsFc3...),** to the actual weight **(PrFc1, PrFc2, PrFc3**...) and to the amount of juice (**PrSFc1**, **PrSFc2, PrSFc3**...) extracted therefrom;
- calculating, based on said estimated volume values **(VsFc1, VsFc2, VsFc3...)** and actual weight (**PrSFc1**, **PrSFc2, PrSFc3...)**, a density value **(DcFc1, DcFc2, DcFc3...);**
- calculating, based on said estimated volume values **(VsFc1, VsFc2, VsFc3...)** and/or actual weight (**PrSFc1**, **PrSFc2, PrSFc3**...) and extracted amount of juice of juice (**PrSFc1**, **PrSFc2, PrSFc3...**), a value relating to the extracted amount of juice **(%Fc1, %Fc2, %Fc3**...).

4. Method according to claim 3, further comprising the following steps:
- collecting third data relating to all pairs of data **(DcFc1, %Fc1; DcFc2, %Fc2; DcFc3, %Fc3...)** consisting of the calculated density **(DcFc1, DcFc2, DcFc3...)** and the calculated value relating to the extracted amount of juice **(%Fc1, %Fc2, %Fc3...)** relating to said predetermined number of sample juice fruits **(Fc1, Fc2, Fc3...);**
- calculating the linear regression of said pairs of calibration data **(DcFc1, %Fc1; DcFc2, %Fc2; DcFc3, %Fc3...)** to obtain said at least one first plurality **(52)** of pairs of reference data **(Dr1, %r1; Dr2, %r2; Dr3, %r3...)** for said predetermined batch **(LF).**

5. Method according to claim 3 or 4, wherein the predetermined number of sample juice fruits **(Fc1, Fc2, Fc3...)** is comprised between 40 and 80.

6. Method according to one or more of the preceding claims, wherein said value relating to an estimated amount of juice **(%sF)** for said at least one juice fruit **(F)** and/or said calculated value relating to the extracted amount of juice **(%Fc1, %Fc2, %Fc3...)** is the estimated by weight percentage value of juice present in said at least one juice fruit **(F)** with respect to its actual weight **(PrF)** and/or the actual by weight percentage value of juice extracted from each of the sample juice fruits **(Fc1, Fc2, Fc3...)** with respect to its actual weight (**PrSFc1**, **PrSFc2, PrSFc3...).**

7. A computer program for the non-destructive determination of the juice content of at least one juice fruit, comprising instructions which, once run by a processor, command the processor to carry out at least the steps for calculating the density **(DcF)** and processing a value relating to an estimated amount of juice **(%sF)** for said at least one juice fruit **(F)** of the method according to one or more of the preceding claims.

8. A program according to claim 7, further comprising instructions which, once run by a processor, command the processor to further carry out the steps of:
- for each sample juice fruit **(Fc1, Fc2, Fc3...),** calculating the density value **(DcFc1, DcFc2, DcFc3...),** of the value relating to the extracted amount of juice **(%Fc1, %Fc2, %Fc3...)** and association thereof, so as to create a plurality of pairs of calibration data **(DcFc1, %Fc1; DcFc2, %Fc2; DcFc3, %Fc3...);**
- calculating the linear regression of the pairs of calibration data **(DcFc1, %Fc1; DcFc2, %Fc2; DcFc3, %Fc3...)** to obtain said at least one first plurality **(52)** of pairs of reference data **(Dr1, %r1; Dr2, %r2; Dr3, %r3...)** for said predetermined batch **(LF);** of the method according to one or more of claims 4 to 6.

9. A computer system for the non-destructive determination of the juice content of at least one juice fruit, comprising:
- a data collection unit configured to collect at least the following data:
- first data relating to the estimated volume **(VsF)** and the actual weight **(PrF)** of said at least one juice fruit **(F);**
- at least one first plurality of pairs of reference data **(Dr1, %r1; Dr2, %r2; Dr3, %r3...)** for said at least one juice fruit, each pair of reference data consisting of a reference density value **(Dr1; Dr2; Dr3...)** and a corresponding value relating to a reference amount of juice **(%r1; %r2; %r3...);**
- a microprocessor unit operatively connected or connectable to said data collection unit;
wherein said microprocessor unit is configured to run the computer program according to the preceding claim.

10. An assembly for the non-destructive determination of the juice content of at least one juice fruit, comprising:
- scanning means **(20)** configured to determine the estimated volume **(VsF)** of said at least one juice fruit **(F);**
- weighing means **(30)** for determining the actual weight **(PrF)** of said at least one juice fruit **(F);**
- a computer system according to the preceding claim **(50),** preferably operatively connected with said scanning means **(20)** and with said weighing means **(30).**

11. The use of the assembly according to the preceding claim for the quality classification of said at least one juice fruit **(F)** based on the value relating to the estimated amount of juice **(%sF)** by means of this assembly.

## Patentansprüche

1. Computerimplementiertes Verfahren für die zerstörungsfreie Bestimmung des Saftgehalts wenigstens einer Saftfrucht, umfassend wenigstens die folgenden Schritte:
- Sammeln erster Daten in Bezug auf das geschätzte Volumen (VsF) und das tatsächliche Gewicht (PrF) der wenigstens einen Saftfrucht (F);
- Sammeln wenigstens einer ersten Vielzahl von Paaren von Referenzdaten (Dr1, %r1; Dr2, %r2; Dr3, %r3 ...) für die wenigstens eine Saftfrucht, wobei jedes Paar von Referenzdaten aus einem Referenz Dichtewert (Dr1; Dr2; Dr3...) und einem entsprechenden Wert in Bezug auf die Referenzmenge von Saft (%r1; %r2; %r3 ...) besteht;
- Berechnen der Dichte (DcF) der wenigstens einen Saftfrucht (F) basierend auf den ersten Daten (VsF, PrF);
- Verarbeiten eines Werts bezogen auf einen geschätzten Anteil von Saft (%sF) für die wenigstens eine Saftfrucht (F) basierend auf der wenigstens einen ersten Vielzahl von Paaren von Referenzdaten (Dr1, %r1; Dr2, %r2; Dr3, %r3 ...) und dem berechneten Dichtewert (%sF).

2. Verfahren nach Anspruch 1, bei welchem die wenigstens eine Saftfrucht (F) eine vorbestimmte, bekannte Qualität aufweist und die wenigstens eine erste Vielzahl von Paaren von Referenzdaten (Dr1, %r1; Dr2, %r2; Dr3, %r3 ...) sich auf die vorbestimmte bekannte Qualität bezieht.

3. Verfahren nach Anspruch 1, das dazu geeignet ist, den Saftgehalt von Saftfrüchten (F) zu bestimmen, die zu einem vorbestimmten Los (LF) gehören, wobei die Referenzdaten (Dr1, %r1; Dr2, %r2; Dr3, %r3 ...) ausgehend von einer vorbestimmten Anzahl von Muster-Saftfrüchten (Fc1, Fc2, Fc3...) bestimmt werden, die zu dem vorbestimmten Los (LF) gehören, wobei das Verfahren weiterhin für jede Muster-Saftfrucht (Fc1, FC2, Fc3...) wenigstens die folgenden Schritte umfasst:
- Sammeln zweiter Daten, die sich auf ein geschätztes Volumen (VsFc1, VsFc2, VsFc3...), das tatsächliche Gewicht (PrFc1, PrFc2, PrFc3...) und die Menge des daraus extrahierten Safts (PrSFc1, PrSFc2, PrSFc3...) beziehen;
- Berechnen eines Dichtewerts (DcFc1, DcFc2, DcFc3...) basierend auf den geschätzten Volumenwerten (VsFc1, VsFc2, VsFc3...) und dem tatsächlichen Gewicht (PrFc1, PrFc2, PrFc3...);
- Berechnen eines Werts betreffend die extrahierte Menge von Saft (%Fc1, %Fc2, %Fc3) basierend auf den geschätzten Volumenwerten (VsFc1, VsFc2, VsFc3...) und/oder dem tatsächlichen Gewicht (PrSFc1, PrSFc2, PrSFc3) und dem extrahierten Saft.

4. Verfahren nach Anspruch 3, weiterhin umfassend die folgenden Schritte:
- Sammeln dritter Daten, die sich auf alle Datenpaare (DCFC1, %Fc1; DCFc2, %Fc2; DcFc3, %Fc3...) beziehen, die aus der berechnenden Dichte (DCF1, DcFc2. DcFc3...) und dem berechneten Wert bezüglich der extrahierten Menge des Safts (%Fc1, %Fc2, &FC3...) bezogen auf die vorbestimmte Anzahl der Muster Saftfrüchte (FC1, Fc2, Fc3...) bestehen;
- Berechnen der linearen Regression der Paare von Kalibrierungsdaten (DcFc1, %Fc1; DcFc2, %Fc2; DcFc3, %Fc3...), um die wenigstens eine erste Vielzahl (52) von Paaren von Referenzdaten (Dr1, %r1; Dr2, %r2; Dr3, %r3...) für das vorbestimmte Los (LF) zu erhalten.

5. Verfahren nach Anspruch 3 oder 4, bei welchem die vorbestimmte Anzahl von Mustersaftfrüchten (Fc1, FC2, Fc3...) zwischen 40 und 80 ist.

6. Verfahren nach einem oder mehreren der vorhergehenden Ansprüche, bei welchem der Wert, der sich auf die geschätzte Anzahl von Saft (%sF) für die wenigstens eine Saftfrucht (F) und/oder den berechneten Wert, der sich auf die extrahierte Menge von Saft (%Fc1, %Fc2, %Fc3...) bezieht, der geschätzte Gewichtsprozent Wert des in der wenigstens einen Saftfrucht (F) enthaltenen Safts bezüglich deren tatsächlichen Gewichts (PrF) und/oder der tatsächliche Gewichtsprozent Anteil des Safts ist, der aus jeder der Muster-Saft Früchte (Fc1, Fc2, Fc3...) bezogen auf deren tatsächliches Gewicht (PrSFc1, PrSFc2, PrSfc3...) extrahiert wurde.

7. Computerprogramm zur zerstörungsfreien Bestimmung des Saftinhalts wenigstens einer Saftfrucht, umfassend Befehle, die bei Ausführung durch einen Prozessor den Prozessor dazu veranlassen, wenigstens einen der Schritte zur Berechnung der Dichte (DcF) durchzuführen und einen Wert zu verarbeiten, der sich auf den geschätzten Anteil von Saft (%sF) der wenigstens einen Saftfrucht (F) gemäß dem Verfahren nach einem oder mehrerer der vorhergehenden Ansprüche bezieht.

8. Verfahren nach Anspruch 7, weiterhin umfassend Befehle, welche, wenn diese durch einen Prozessor ausgeführt werden, den Prozessor dazu veranlassen, weiterhin folgende Schritte auszuführen:
- Berechnen des Dichtewerts (DcFc1, DcFc2, DcFc3...) für jede Mustersaftfrucht (Fc1, Fc2, Fc3...), aus dem Wert, der auf die extrahierte Menge von Saft (%Fc1, %Fc2, %Fc3...) bezogen ist, und dessen Zuordnung, um eine Vielzahl von Paaren von Kalibrierungsdaten (DcFc1, %Fc1; DcFc2, %Fc2; DcFc3, %Fc3...) zu erzeugen;
- Berechnen der linearen Regression der Paare von Kalibrierungsdaten (DcFc1, %Fc1; DcFc2, %Fc2; DcFc3, %Fc3...), um wenigstens eine erste Vielzahl (52) von Paaren von Referenzdaten (Dr1, %r1; Dr2, %r2; Dr3, %r3 ...) für das vorbestimmte Los (LF) zu erhalten; gemäß dem Verfahren nach einem oder mehrerer der Ansprüche 4 bis 6.

9. Computersystem für die zerstörungsfreie Bestimmung des Saftgehalts wenigstens einer Saftfrucht, umfassend:
- Eine Datensammlungseinheit, die dazu ausgebildet ist, wenigstens die folgenden Daten zu sammeln:
- Erste Daten, die sich auf das geschätzte Volumen (VsF) und das tatsächliche Gewicht (PrF) der wenigstens einen Saftfrucht (F) beziehen;
- Wenigstens eine erste Vielzahl von Paaren von Referenzdaten (Dr1, %r1; Dr2, %r2; Dr3, %r3...) für die wenigstens eine Saftfrucht, wobei jedes Paar von Referenzdaten aus einem Referenz Dichtewert (Dr1; Dr2; Dr3...) und einem entsprechenden Wert bezogen auf einer Referenzmenge an Saft (%r1; %r2; %r3...) besteht;
- eine Mikroprozessoreinheit, die in Wirkverbindung mit der Daten Sammeleinheit ist oder mit dieser in Wirkverbindung bringbar ist;
wobei die Mikroprozessoreinheit ausgebildet ist, das Computerprogramm gemäß dem vorstehenden Anspruch auszuführen.

10. Anordnung für die zerstörungsfreie Bestimmung des Saftgehalts wenigstens einer Saftfrucht, umfassend:
- Scan Mittel (20), die dazu ausgebildet sind, das geschätzte Volumen (VsF) der wenigstens einen Saftfrucht (F) zu bestimmen;
- Wiegemittel (30) zur Bestimmung des tatsächlichen Gewichts (PrF) der wenigstens einen Saftfrucht (F);
- ein Computersystem (50) nach dem vorhergehenden Anspruch, vorzugsweise in Wirkverbindung mit den Scan Mitteln (20) und mit den Wiegemitteln (30).

11. Verwendung einer Anordnung nach dem vorhergehenden Anspruch zur Qualitätsklassifizierung wenigstens einer Saftfrucht (F) basierend auf dem Wert betreffend die geschätzte Saftmenge (%sF) mittels dieser Anordnung.

## Revendications

1. Un procédé mis en œuvre par ordinateur pour la détermination non destructive du contenu du jus d'au moins un fruit à jus, comprenant au moins les étapes suivantes :
- la collecte de premières données concernant le volume estimé (VsF) et le poids réel (PrF) dudit au moins un fruit à jus (F) ;
- la collecte d'au moins une première pluralité de paires de données de référence (Dr1, %r1 ; Dr2, %r2 ; Dr3, %r3...) pour ledit au moins un fruit à jus, chaque paire de données de référence consistant en une valeur de densité de référence (Dr1 ; Dr2 ; Dr3...) et une valeur correspondante concernant la quantité de référence de jus (%r1 ; %r2 ; %r3...) ;
- le calcul, sur la base desdites premières données (VsF, PrF), de la densité (DcF) dudit au moins un fruit à jus (F) ;
- le traitement, sur la base de ladite au moins une première pluralité de paires de données de référence (Dr1, %r1 ; Dr2, %r2 ; Dr3, %r3...) et de ladite valeur de densité calculée (DcF), d'une valeur concernant une quantité estimée de jus (%sF) pour ledit au moins un fruit à jus (F).

2. Procédé selon la revendication 1, dans lequel ledit au moins un fruit à jus (F) est d'une qualité connue prédéterminée, ladite au moins une première pluralité de paires de données de référence (Dr1, %r1 ; Dr2, %r2 ; Dr3, %r3...) concernant ladite qualité connue prédéterminée.

3. Procédé selon la revendication 1, adapté pour déterminer le contenu du jus des fruits à jus (F) appartenant à un lot prédéterminé (LF), lesdites données de référence (Dr1, %r1 ; Dr2, %r2 ; Dr3, %r3...) étant déterminées en commençant à partir d'un nombre prédéterminé de fruits à jus d'échantillons (Fc1, Fc2, Fc3...) appartenant audit lot prédéterminé (LF), le procédé comprenant en outre pour chacun desdits fruits à jus d'échantillons (Fc1, Fc2, Fc3...) au moins les étapes suivantes :
- la collecte de deuxièmes données concernant le volume estimé (VsFc1, VsFc2, VsFc3...), le poids réel (PrFc1, PrFc2, PrFc3...) et la quantité de jus (PrSFc1, PrSFc2, PrSFc3...) extraite de celui-ci ;
- le calcul, sur la base desdites valeurs de volume estimées (VsFc1, VsFc2, VsFc3...) et du poids réel (PrSFc1, PrSFc2, PrSFc3...), d'une valeur de densité (DcFc1, DcFc2, DcFc3...) ;
- le calcul, sur la base desdites valeurs de volume estimées (VsFc1, VsFc2, VsFc3...) et/ou du poids réel (PrSFc1, PrSFc2, PrSFc3...) et de la quantité extraite de jus du jus (PrSFc1, PrSFc2, PrSFc3...), d'une valeur concernant la quantité extraite de jus (%Fc1, %Fc2, %Fc3...).

4. Procédé selon la revendication 3, comprenant en outre les étapes suivantes :
- la collecte de troisièmes données concernant toutes les paires de données (DcFc1, %Fc1 ; DcFc2, %Fc2 ; DcFc3, %Fc3...) consistant en la densité calculée (DcFc1, DcFc2, DcFc3...) et la valeur calculée concernant la quantité extraite de jus (%Fc1, %Fc2, %Fc3...) concernant ledit nombre prédéterminé de fruits à jus d'échantillons (Fc1, Fc2, Fc3...) ;
- le calcul de la régression linéaire desdites paires de données d'étalonnage (DcFc1, %Fc1 ; DcFc2, %Fc2 ; DcFc3, %Fc3...) pour obtenir ladite au moins une première pluralité (52) de paires de données de référence (Dr1, %r1; Dr2, %r2 ; Dr3, %r3...) pour ledit lot prédéterminé (LF).

5. Procédé selon la revendication 3 ou 4, dans lequel le nombre prédéterminé de fruits à jus d'échantillons (Fc1, Fc2, Fc3...) est compris entre 40 et 80.

6. Procédé selon une ou plusieurs des revendications précédentes, dans lequel ladite valeur concernant une quantité estimée de jus (%sF) pour ledit au moins un fruit à jus (F) et/ou ladite valeur calculée concernant la quantité extraite de jus (%Fc1, %Fc2, %Fc3...) est la valeur de pourcentage en poids estimée de jus présent dans ledit au moins un fruit à jus (F) par rapport à son poids réel (PrF) et/ou la valeur de pourcentage en poids réel de jus extrait à partir de chacun des fruits à jus d'échantillons (Fc1, Fc2, Fc3...) par rapport à son poids réel (PrSFc1, PrSFc2, PrSFc3...).

7. Un programme informatique pour la détermination non destructive du contenu du jus d'au moins un fruit à jus, comprenant des instructions qui, une fois exécutées par un processeur, commandent au processeur de mettre en œuvre au moins les étapes de calcul de la densité (DcF) et de traitement d'une valeur concernant une quantité estimée de jus (%sF) pour ledit au moins un fruit à jus (F) du procédé selon une ou plusieurs des revendications précédentes.

8. Un programme selon la revendication 7, comprenant en outre des instructions qui, une fois exécutées par un processeur, commandent au processeur de mettre en œuvre en outre les étapes suivantes :
- pour chaque fruit à jus d'échantillons (Fc1, Fc2, Fc3...), le calcul de la valeur de densité (DcFc1, DcFc2, DcFc3...), de la valeur concernant la quantité extraite de jus (%Fc1, %Fc2, %Fc3...) et d'une association de celles-ci, de façon à créer une pluralité de paires de données d'étalonnage (DcFc1, %Fc1 ; DcFc2, %Fc2 ; DcFc3, %Fc3...) ;
- le calcul de la régression linéaire des paires de données d'étalonnage (DcFc1, %Fc1 ; DcFc2, %Fc2 ; DcFc3, %Fc3...) pour obtenir ladite au moins une première pluralité (52) de paires de données de référence (Dr1, %r1 ; Dr2, %r2 ; Dr3, %r3...) pour ledit lot prédéterminé (LF) du procédé selon une ou plusieurs des revendications 4 à 6.

9. Un système informatique pour la détermination non destructive du contenu du jus d'au moins un fruit à jus, comprenant :
- une unité de collecte de données configurée pour collecter au moins les données suivantes :
- des premières données concernant le volume estimé (VsF) et le poids réel (PrF) dudit au moins un fruit à jus (F) ;
- au moins une première pluralité de paires de données de référence (Dr1, %r1 ; Dr2, %r2 ; Dr3, %r3...) pour ledit au moins un fruit à jus, chaque paire de données de référence consistant en une valeur de densité de référence (Dr1 ; Dr2 ; Dr3...) et une valeur correspondante concernant une quantité de référence de jus (%r1 ; %r2 ; %r3...) ;
- une unité de microprocesseur connectée de manière opérationnelle ou adaptée pour être connectée à ladite unité de collecte de données ;
dans lequel ladite unité de microprocesseur est configurée pour exécuter le programme informatique selon la revendication précédente.

10. Un ensemble pour la détermination non destructive du contenu du jus d'au moins un fruit à jus, comprenant :
- un moyen de balayage (20) configuré pour déterminer la valeur estimée (VsF) dudit au moins un fruit à jus (F) ;
- un moyen de pondération (30) pour déterminer le poids réel (PrF) dudit au moins un fruit à jus (F) ;
- un système informatique selon la revendication précédente (50), connecté de préférence de manière opérationnelle audit moyen de balayage (20) et audit moyen de pondération (30).

11. L'utilisation de l'ensemble selon la revendication précédente pour la classification de qualité dudit au moins un fruit à jus (F) sur la base de la valeur concernant la quantité estimée de jus (%sF) au moyen de cet ensemble.
